# EUROPÄISCHE PATENTSCHRIFT

(11) **EP 1 311 203 B1**
(45) Veröffentlichungstag und Bekanntmachung des Hinweises auf die Patenterteilung: **21.07.2004**
(21) Anmeldenummer: 00962236.6
(22) Anmeldetag: 22.08.2000
(51) Int. Cl.: A61C 1/00, A61L 2/18

(54) **EINRICHTUNG ZUR EINSPEISUNG VON BEHANDLUNGSFLÜSSIGKEIT IN MEDIZINISCHE GERÄTE**
DEVICE FOR FEEDING A TREATMENT LIQUID TO MEDICAL APPLIANCES
DISPOSITIF DESTINE A L'INTRODUCTION DE LIQUIDE DE TRAITEMENT DANS DES APPAREILS MEDICAUX

(43) Veröffentlichungstag der Anmeldung: 21.05.2003
(73) Patentinhaber: Sirona Dental Systems GmbH & Co.KG, 64625 Bensheim (DE)
(72) Erfinder: BEHRINGER, Wolfgang, 74625 Bensheim (DE)
(74) Vertreter: Sommer, Peter
(86) Internationale Anmeldenummer: PCT/DE2000/002861
(87) Internationale Veröffentlichungsnummer: WO 2002/015811

(56) Entgegenhaltungen:
- DE-A- 3 246 266
- DE-C- 19 908 997
- US-A- 5 087 198
- US-A- 5 295 829

## Beschreibung

Die Erfindung betrifft eine Einrichtung zur Einspeisung von Behandlungsflüssigkeit in medizinische Geräte mit einer Austrittsöffnung für die Behandlungsflüssigkeit, insbesondere zur Einspeisung von Wasser in Dentaleinrichtungen, mit einer Zuleitung für die Behandlungsflüssigkeit und mit Mitteln zum Einbringen von Entkeimungsmittel in die Behandlungsflüssigkeit.

Aus der Praxis sind für unterschiedlichste Anwendungen medizinische Geräte bekannt, über die Behandlungsflüssigkeit appliziert wird. Gerade im medizinischen Bereich ist eine Verkeimung der Behandlungsflüssigkeit unbedingt zu vermeiden. Die in diesem Zusammenhang auftretenden Probleme werden nachfolgend am Beispiel einer zahnärztlichen Behandlungseinheit, einer sogenannten Dentaleinrichtung, mit einer Kühl- und Spülflüssigkeitsversorgung erläutert.

Dentaleinrichtungen verwenden in der Regel Trinkwasser als Kühl- und Spülflüssigkeit, das dem öffentlichen Trinkwassernetz entnommen wird. Schon im Anschlußbereich Trinkwassernetz-/Dentaleinrichtung werden häufig sehr hohe, weit über der von der Trinkwasserverordnung festgesetzten Grenze von 100 KBE/ml liegende Keimzahlen gefunden und auch des öfteren Problemkeime, wie "Pseudomonas aeruginosa" oder "Legionellen", nachgewiesen. Zudem weisen zahnärztliche Behandlungseinheiten gegenüber dem Trinkwassersystem einige Besonderheiten auf, welche das Bakterienwachstum und die dadurch entstehenden hohen Keimzahlen begünstigen. Zahnärztliche Behandlungseinheiten umfassen in der Regel mehrere Handstücke mit unterschiedlichen Behandlungsaufsätzen, wobei jedes Handstück an eine eigene Zapfstelle angeschlossen ist. Bedingt durch eine unterschiedliche Nutzung der einzelnen Handstücke, durch Behandlungspausen und auch die Stagnation des Wassers über das Wochenende kommt es häufig zu relativ langen Verweildauern des Wassers in der Behandlungseinheit. Dabei erwärmt sich das stehende Wasser auf Raumtemperatur. Außerdem wird das Wasser vor der Applikation auf Körpertemperatur erwärmt. Das Keimwachstum wird ferner durch die englumigen Schläuche, Querschnittsverengungen und Totraumbildung, z. B. in Ventilen, begünstigt. Hinzu kommt, dass die meisten wasserführenden Bauteile und auch die Schläuche zahnärztlicher Behandlungseinheiten aus Kunststoff gebildet sind, so daß sie auch wegen der großen Oberfläche pro Volumen Wasser einen idealen Nährboden für das Keimwachstum bilden.

Eine Entkeimung des Leitungswassers vor der Applikation ist daher ratsam, wenn nicht sogar unbedingt erforderlich. Dazu werden in der Praxis Entkeimungsanlagen oder -zollen angesetzt, die dem Leitungswasser entweder ein geeignetes Entkeimungsmittel zudosieren oder, beispielsweise auf elektrolytischem Wege, erzeugen.

Es hat sich jedoch gezeigt, dass das Keimwachstum mit Hilfe der bekannten Entkeimungsanlagen nicht vollständig unterbunden werden kann, sondern mit dem Leitungsabstand von der Entkeimungsanlage zunimmt. Insgesamt läßt sich mit Hilfe der bekannten Entkeimungsanlagen eine Wiederverkeimung nicht zuverlässig verhindern.

Deshalb bieten einige Hersteller von Dentaleinrichtungen sogenannte Sanierungsprogramme an. Im Rahmen dieser Sanierungsprogramme wird hochkonzentriertes Entkeimungsmittel durch die gesamte Dentaleinrichtung, durch jede Zapfstelle und jedes Handstück gepumpt und für einige Stunden in den Leitungen der Dentaleinrichtung belassen. Danach wird die Dentaleinrichtung so lange mit Leitungswasser gespült, bis die Konzentration des Entkeimungsmittels so gering ist, daß sie für einen Patienten unschädlich ist.

Die Durchführung eines Sanierungsprogramms, wie es voranstehend beschrieben ist, dauert in der Regel 12 - 24 Stunden, innerhalb derer die Dentaleinrichtung dann nicht verwendet werden kann. Da bislang keine Möglichkeit besteht, den Verkeimungsgrad der Dentaleindchtung zu bestimmen, um zu erkennen, ob eine Sanierung tatsächlich nötig ist, kann eine Wiederverkeimung nur durch regelmäßige Anwendung des voranstehend beschriebenen Sanierungsprogramms wirkungsvoll verhindert werden.

Aus der deutschen Offenlegungsschrift 32 46 266 und aus der deutschen Offenlegungsschrift 36 35 568 sind Einrichtungen zur Desinfektion von Wasserwegen in zahnmedizinischen Geräten bekannt, die ein periodisches Befluten der Wasserwege mit einem Desinfektionsmittel vorsehen. In beiden Fällen sind Zuleitungen für die Behandlungsflüssigkeit und Mittel zum Einbringen von Entkeimungsmittel in die Behandlungsflüssigkeit vorgesehen. Der Austrittsöffnung in der Zuleitung der bekannten Vorrichtungen sind Ventile zugeordnet.

Gegenstand der deutschen Offenlegungsschrift 195 09 180 A1 ist eine Vorrichtung zum Reinigen wenigstens eines Medienkanals und eines sogenannten Antriebskanals in einen medizinischen, insbesondere zahnärztlichen Handstück. Bei dieser Vorrichtung sind jeweils Ventile für die Zuführungsleitungen vorgesehen. Außerdem ist in einer Verbindungsleitung ein besonderes Rückschlagventil angeordnet.

Gegenstand des US-Patents 5 087 198 ist ein zahnmedizinisches Gerät, in das ebenfalls Behandlungsflüssigkeit eingespeist werden kann. Dieses Gerät umfaßt stromaufwärts Dreiwegeventile zur Umschaltung von Behandlungsflüssigkeiten.

Aus der EP 0 524 334 A1 ist eine Wasserversorgungseinrichtung für einen zahnärztlichen Arbeitsplatz gemäß dem Oberbegriff von Anspruch 1 bekannt, der mehrere Entnahmestellen aufweisende Gerätschaften enthält, denen Wasser aus dem Trinkwasser- Leitungsnetz zugeführt wird. Über eine Pumpe wird Wasser mit dem erforderlichen Druck und dem notwendigen Volumen aus einem Behälter zu einer Entnahmestelle gefördert. Dabei kann Desinfektionsflüssigkeit zugeführt werden.

Der vorliegenden Erfindung liegt nun die Aufgabe zugrunde, eine Einrichtung zur Einspeisung von Behandlungsflüssigkeit in medizinische Geräte der eingangs genannten Art anzugeben, mit der sich eine Verkeimung bzw. eine Wiederverkeimung einfach aber wirkungsvoll verhindern läßt.

Die erfindungsgemäße Einrichtung löst die voranstehende Aufgabe durch die Merkmale des Patentanspruchs 1. Danach ist die eingangs genannte Einrichtung so ausgestaltet, daß der Austrittsöffnung ein Umschaltventil vorgeschaltet ist, an das die Zuleitung angeschlossen ist, und daß eine Rückspülleitung mit einem Rückspülventil an das Umschaltventil angeschlossen ist.

Erfindungsgemäß ist erkannt worden, daß auch mit den bekannten Entkeimungsanlagen eine Wiederverkeimung wirkungsvoll vermieden werden kann, wenn die durch das medizinische Gerät geleitete und mit Entkeimungsmittel versetzte Behandlungsflüssigkeit nicht notwendigerweise über die Austrittsöffnung des medizinischen Geräts freigesetzt wird, sondern alternativ auch zum Durchspülen des medizinischen Gerätes verwendet werden kann. Dazu wird der Austrittsöffnung erfindungsgemäß ein Umschaltventil vorgeschaltet, an das sowohl die Zuleitung als auch eine Rückspülleitung mit einem Rückspülventil angeschlossen sind, so daß die Zuleitung alternativ mit der Austrittsöffnung oder der Rückspülleitung verbunden werden kann. Zum Durchspülen des medizinischen Geräts wird die mit Entkeimungsmittel versetzte Behandlungsflüssigkeit über die Zuleitung bis zu dem Umschaltventil und über das Umschaltventil in die Rückspülleitung geleitet. Dabei ist das Rückspülventil geöffnet.

Da die mit Entkeimungsmittel versetzte Behandlungsflüssigkeit aufgrund der erfindungsgemäß vorgeschlagenen konstruktiven Maßnahmen einfach entweder über die Austrittsöffnung appliziert werden kann oder auch zum Durchspülen verwendet werden kann, besteht nun auch die Möglichkeit, das medizinische Gerät je nach Bedarf, beispielsweise direkt vor der Applikation der Behandlungsflüssigkeit oder auch in Behandlungspausen oder vor der täglichen Inbetriebnahme, mit Behandlungsflüssigkeit durchzuspülen.

Grundsätzlich läßt sich das Ventilkonzept der erfindungsgemäß vorgeschlagenen Einrichtung zur Einspeisung von Behandlungsflüssigkeit mit unterschiedlichen Ventiltypen realisieren.

So könnten das Umschaltventil und das Rückspülventil konstruktiv zusammengefaßt werden, und zwar in einem der Austrittsöffnung vorgeschalteten Drei-Wege-Ventil, mit dem wahlweise entweder die Austrittsöffnung oder die Rückspülleitung an die Zuleitung angeschlossen werden kann.

Bei den meisten medizinischen Geräten und insbesondere auch bei Dentaleinrichtungen ist gerade im Bereich der Austrittsöffnung, in dem auch das Umschaltventil angeordnet sein sollte, eine möglichst kleine Baugröße anzustreben. Dazu erweist es sich als vorteilhaft, das Rückspülventil in der Rückspülleitung, also abgesetzt vom Umschaltventil, anzuordnen. In diesem Falle kann als Umschaltventil ein druckgesteuertes Ventil verwendet werden, das in Abhängigkeit vom Schaltzustand des Rückspülventils in der Rückspülleitung und der dadurch bedingten Druckverhältnisse in der Rückspülleitung öffnet bzw. schließt. Im Gegensatz zu anderen Ventilformen, wie z.B. elektrisch geschalteten Ventilen, sind hier keine zusätzlichen Steuermittel, wie Drähte etc., erforderlich, die sich negativ auf die Baugröße des Umschaltventils auswirken würden.

Als besonders vorteilhaft erweist es sich, das Umschaltventil als Rückschlagventil zu realisieren. Auf diese Weise kann nämlich einfach ein Rücksaugen über die Austrittsöffnung und ein damit verbundenes Eindringen von Flüssigkeit und Bakterien in das medizinische Gerät verhindert werden.

Wie bereits erwähnt, umfaßt die erfindungsgemäße Einrichtung zur Einspeisung von Behandlungsflüssigkeit in medizinische Geräte Mittel zum Einbringen von Entkeimungsmittel in die Behandlungsflüssigkeit, wobei es sich hierbei um die aus der Praxis bekannten Entkeimungsanlagen handeln kann. In einer besonders vorteilhaften Variante der erfindungsgemäßen Einrichtung sind zusätzlich Mittel zur Bestimmung der Art und/oder der Menge der in der Behandlungsflüssigkeit bzw. in dem medizinischen Gerät vorliegenden Keime vorgesehen, so daß sich die Art und Dosierung des beizumischenden Entkeimungsmittels sowie die Rückspülzeit individuell bestimmen lassen. Des Weiteren können Mittel zur Bestimmung der Konzentration und ggf. der Art des Entkeimungsmittels in der Behandlungsflüssigkeit vorgesehen sein, so daß sich die Dosierung des der Behandlungsflüssigkeit beigemischten Entkeimungsmittels überwachen und ggf. auch regeln läßt. Damit läßt sich beispielsweise einfach feststellen, ob das Entkeimungsmittel während einer Stillstandzeit durch Zehrung aufgebraucht worden ist. Falls auch Mittel zum Regeln der Konzentration des Entkeimungsmittels in der Behandlungsflüssigkeit vorhanden sind, kann das medizinische Gerät auch gezielt mit Behandlungsflüssigkeit durchgespült werden, der Entkeimungsmittel in höherer Konzentration beigemischt ist als für die Applikation vorgesehen.

Als vorteilhaft erweist es sich ferner, einen Vorratsbehälter für die Behandlungsflüssigkeit vorzusehen und die Behandlungsflüssigkeit mit Hilfe einer Pumpe aus diesem Vorratsbehälter zu fördern. In diesem Falle kann die Behandlungsflüssigkeit mit Hilfe einer dem Vorratsbehälter zugeordneten Dosiervorrichtung für mindestens ein Entkeimungsmittel bereits im Vorratsbehälter mit Entkeimungsmittel versetzt werden, so dass auf eine zusätzliche, in der Zuleitung angeordnete Entkeimungsanlage verzichtet werden kann. Eine zusätzliche, dem Vorratsbehälter vorgeschaltete Entkeimungsanlage hätte den Vorteil, dass auch der Vorratsbehälter und die Pumpe entkeimt würden. Eine derartig angeordnete Entkeimungsanlage könnte auch alternativ zu einer dem Vorratsbehälter zugeordneten Dosiervorrichtung verwendet werden.

Bei der erfindungsgemäßen Einrichtung ist die Rückspülleitung über eine Abflußventil alternativ an einen Abfluß oder an den Vorratsbehälter anschließbar, so dass die Behandlungsflüssigkeit beim Durchspülen des medizinischen Geräts alternativ in den Abfluß gepumpt werden kann oder im Kreislauf vom Vorratsbehälter über die Zuleitung zum Umschaltventil und über die Rückspülleitung wieder in den Vorratsbehälter gepumpt werden kann. Die Möglichkeit, die mit Entkeimungsmittel versetzte Behandlungsflüssigkeit im Kreislauf durch die Einrichtung und das medizinische Gerät zu pumpen erweist sich insbesondere dann als vorteilhaft, wenn eine Durchspülvorgang mit höher konzentriertem Entkeimungsmittel erforderlich ist.

Der Vorratsbehälter sollte sich vorteilhafter Weise auch direkt entleeren lassen, beispielweise um Ablagerungen im Vorratsbehälter auszuspülen. In einer vorteilhaften Variante der erfindungsgemäßen Einsichtung könnte dazu ein Ablaßventil vorgesehen sein.

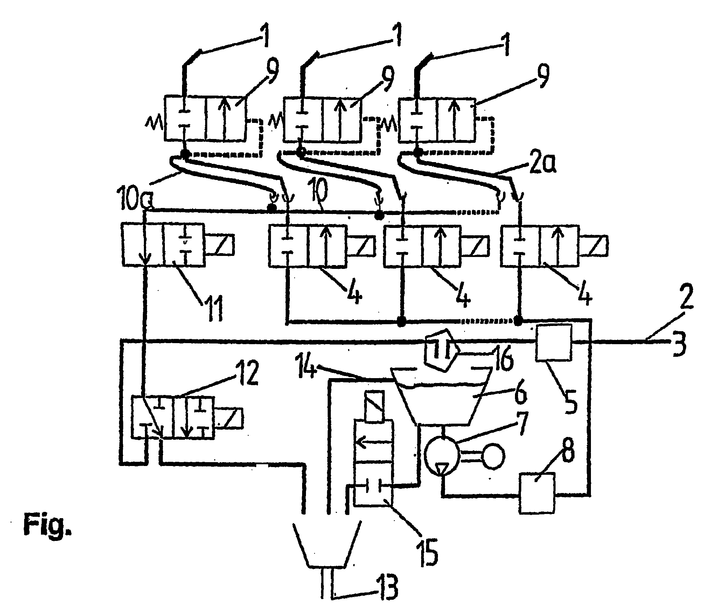

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung eines Ausführungsbeispiels der Erfindung anhand der Zeichnung zu verweisen.

Die einzige Figur zeigt in schematischer Darstellung eine erfindungsgemäße Einrichtung zur Einspeisung von Wasser in eine zahnärztliche Behandlungseinheit mit drei Handstücken für unterschiedliche Dentalinstrumente 1. Jedes dieser Dentalinstrumente 1 weist eine Austrittsöffnung auf, über die sich während der Behandlung eines Patienten Wasser als Kühl- und/oder Spülflüssigkeit applizieren läßt. Dementsprechend müssen die Dentalinstrumente 1 zumindest während der Behandlung eines Patienten mit Wasser versorgt werden.

Die in der einzigen Figur nicht näher bezeichnete zahnärztliche Behandlungseinheit ist dazu über eine Zuleitung 2 an das öffentliche Trinkwasser-Leitungsnetz 3 angeschlossen, und die einzelnen Handstücke bzw. Dentalinstrumente 1 sir.d parallel, über zugeordnete Anschlußventile 4 und zuleitungsabschnitte 2a an die Zuleitung 2 angeschlossen.

Im hier dargestellten Ausführungsbeispiel wird das aus dem Leitungsnetz 3 entnommene Trinkwasser zunächst über eine in der Zuleitung 2 angeordnete Entkeimungsanlage 5, wo es mit einem Entkeimungsmittel versetzt wird, in einen Vorratsbehälter 6 geleitet. Von dort wird es mit Hilfe einer Pumpe 7 über eine weitere Entkeimungsanlage 8 den einzelnen Dentalinstrumenten 1 zugeleitet.

Erfindungsgemäß ist der Austrittsöffnung jedes Dentalinstruments 1 ein Umschaltventil 9 vorgeschaltet, an das die Zuleitung 2 bzw. der jeweilige zuleitungsabschnitt 2a angeschlossen ist. Neben der Zuleitung 2 ist eine Rückspülleitung 10a, 10 mit einem Rückspülventil 11 an das Umschaltventil 9 angeschlossen.

Im hier dargestellten Ausführungsbeispiel befinden sich die Umschaltventile 9 der einzelnen Dentalinstrumente 1 jeweils sehr nahe an der Austrittsöffnung, so daß jedem Dentalinstrument 1 ein eigener Rückspülleitungsabschnitt 10a zugeordnet ist, der parallel zum jeweiligen Zuleitungsabschnitt 2a im zugeordneten Handstückschlauch geführt ist. Die Rückspülleitungsabschnitte 10a der einzelnen Dentalinstrumente 1 sind dann an eine gemeinsame Rückspülleitung 10 angeschlossen, in der auch das Rückspülventil 11 angeordnet ist. Alternativ dazu könnte auch in jedem Rückspülleitungsabschnitt 10a ein dem jeweiligen Dentalinstrument zugeordnetes Rückspülventil angeordnet sein.

Die Umschaltventile 9 der einzelnen Dentalinstrumente 1 sind hier als Rückschlagventile ausgelegt, so daß ein Ansaugen von Flüssigkeit durch die Austrittsöffnung eines Dentalinstruments 1 und ein damit verbundenes Eindringen von Bakterien in die Behandlungseinheit unterbunden wird. Außerdem sind die Umschaltventile 9 im hier dargestellten Ausführungsbeispiel alle druckgesteuert. Der Schaltzustand der Umschaltventile 9 hängt hier also einerseits vom Schaltzustand des dem jeweiligen Dentalinstrument 1 zugeordneten Anschlußventils 4 ab und andererseits vom Schaltzustand des Rückspülventils 11. Zum Einsatz eines der drei vorhandenen Dentalinstrumente wird zunächst das zugehörige Anschlußventil 4 geöffnet. Bei gleichzeitig geschlossenem Rückspülventil 11 entsteht ein Rückstaudruck in der Rückspülleitung 10 bzw. im Rückspülleitungsabschnitt 10a des Dentalinstruments 1, der wiederum ein Öffnen des zugeordneten Umschaltventils 9 bewirkt, so dass Wasser über die Austrittsöffnung des Dentalinstruments 1 appliziert werden kann. Wird das Rückspülventil 11 bei geöffnetem Anschlußventil 4 ebenfalls geöffnet, dann schließt das Umschaltventil 9 automatisch. Das mit Entkeimungsmittel versetzte Wasser wird dann über die Zuleitung 2 und das Anschlußventil 4 in das Dentalinstrument 1 bis zum Umschaltventil 9 gepumpt und strömt von dort über die Rückspülleitung 10a, 10, das Rückspülventil 11 und ein Abflußventil 12 alternativ entweder in einen Abfluß 13 oder - je nach Schaltstellung des Abflußventils 12 - zurück in den Vorratsbehälter 6, von wo es im Kreislauf wieder zu dem Dentalinstrument 1 und zurück in den Vorratsbehälter 6 gepumpt werden kann. Dieser Durchspülvorgang kann dann beliebig oft wiederholt werden, indem das mit Entkeimungsmittel versetzte Versorgungswasser ständig oder in vorgegebenen Intervallen durch das Leitungssystem der Behandlungseinheit gepumpt wird.

Der Vorratsbehälter 6 ist im hier dargestellten Ausführungsbeispiel noch mit einem Überlauf 14 versehen, der ebenfalls dem Abfluß 13 zugeführt wird. Über ein Ablaßventil 15 kann der Vorratsbehälter 6 direkt in den Abfluß 13 entleert werden.

Schließlich ist in der Zuleitung 2 zwischen der Entkeimungsanlage 5 und dem Vorratsbehälter 6 noch eine Meßsonde 16 angeordnet, die zur Bestimmung der Konzentration und ggf. der Art des Entkeimungsmittels in der Behandlungsflüssigkeit dient, so dass sich die Dosierung des der Behandlungsflüssigkeit beigemischten Entkeimungsmittels überwachen und ggf. auch regeln läßt. Bei entsprechender Auslegung könnte die Meßsonde 16 auch zur Bestimmung der Art und/oder Menge der vorliegenden Keime vorgesehen sein, um die Art und Dosierung des beizumischenden Entkeimungsmittels sowie die Rückspülzeit individuell bestimmen zu können.

Zum Durchspülen des gesamten Leitungssystems nach dem Einschalten der Behandlungseinheit wird zunächst das Rückspülventil 11 geöffnet. Danach wird das der Entkeimungsanlage 8 entfernteste Anschlußventil 4 geöffnet, um die Zuleitung 2, den ,Zuleitungsabschnitt 2a und den Rückspülleitungsabschnitt 10a des der Entkeimungsanlage 8 entferntesten Dentalinstruments 1 sowie die Rückleitung 10 mit dem Rückspülventil 11 ausreichend lange durchzuspülen. Nun wird das Anschlußventil 4 des ersten Dentalinstruments 1 wieder geschlossen und das Anschlußventil 4 des nächsten Dentalinstruments 1 geöffnet. Bei diesem Durchspülvorgang muß nur noch das Volumen in dem zugehörigen Zuleitungsabschnitt 2a und Rückspülleitungsabschnitt 10a ausgetauscht werden. Der Vorgang wird solange fortgesetzt, bis die Zuleitungsabschnitte 2a und Rückspülleitungsabschnitte 10a aller Dentalinstrumente 1 gespült sind. Dadurch wird sichergestellt, daß das gesamte Leitungssystem mit Entkeimungsmittel versorgt ist. Der voran stehend beschriebene Durchspülvorgang wird bei allen Handstücken bzw. Dentalinstrumenten, wiederholt, die in einer dem Entkeimungsmittel angepaßten Stillstandszeit nicht ausreichend lange in Betrieb waren.

Mit der voranstehend beschriebenen Einrichtung läßt sich auf einfache Weise aber dennoch zuverlässig das Keimwachstum im gesamten Leitungssystem der Behandlungseinheit verhindern, indem das gesamte Leitungssystem mit Behandlungsflüssigkeit gespült wird, der Entkeimungsmittel in relativ geringen Dosen beigemischt ist.

## Patentansprüche

1. Einrichtung zur Einspeisung von Behandlungsflüssigkeit in medizinische Geräte mit einer Austrittsöffnung für die Behandlungsflüssigkeit, insbesondere Zur Einspeisung von Wasser in Dentaleinrichtungen,
- mit einer Zuleitung (2) für die Behandlungsflüssigkeit
- mit Mitteln zum Einbringen von Entkeimungsmittel in die Behandlungsflüssigkeit und
- mit einem der Austrittsöffnung vorgeschalteten Umschaltventil (9), an das die Zuleitung (2) angeschlossen ist,
- wobei ein Vorratsbehälter (6) für die Behandlungsflüssigkeit vorgesehen ist und wobei die Behandlungsflüssigkeit mit Hilfe einer Pumpe (7) aus dem Vorratsbehälter (6) gefördert wird,
**dadurch gekennzeichnet, dass** eine Rückspülleitung (10) mit einem Rückspülventil (11) an das Umschaltventil (9) angeschlossen ist und dass die Rückspülleitung (10) über ein Abflußventil (12) alternativ an einen Abfluß (13) oder an den Vorratsbehälter (6) anschließbar ist, so dass die Behandlungsflüssigkeit alternativ in den Abfluß (13) gepumpt oder im Kreislauf vom Vorratsbehälter (6) über die Zuleitung (2) zum Umschaltventil (9) und über die Rückspülleitung (10) wieder in den Vorratsbehälter (6) gepumpt wird.

2. Einrichtung nach Anspruch 1, **dadurch gekennzeichnet, dass** das Umschaltventil (9) und das Rückspülventil (11) in einem der Austrittsöffnung vorgeschalteten Drei-Wege-Ventil realisiert sind.

3. Einrichtung nach Anspruch 1, **dadurch gekennzeichnet, dass** das Rückspülventil (11) in der Rückspülleitung (10) angeordnet ist und dass das Umschaltventil (9) druckgesteuert ist, so dass es bei geschlossenem Rückspülventil (11) aufgrund des sich in der Rückspülleitung (10) aufbauenden Staudrucks öffnet und bei geöffnetem Rückspülventil (11) schließt.

4. Einrichtung nach einem der Ansprüche 1 bis 3, **dadurch gekennzeichnet, dass** das Umschaltventil (9) als Rückschlagventil realisiert ist.

5. Einrichtung nach einem der Ansprüche 1 bis 4, **dadurch gekennzeichnet, dass** Mittel zur Bestimmung der Art und/oder Menge der vorliegenden Keime vorgesehen sind, so dass sich die Art und Dosierung des beizumischenden Entkeimungsmittels sowie die Rückspülzeit individuell bestimmen lassen.

6. Einrichtung nach einem der Ansprüche 1 bis 5, **dadurch gekennzeichnet, dass** Mittel zur Bestimmung der Konzentration und ggf. der Art des Entkeimungsmittels in der Behandlungsflüssigkeit vorgesehen sind, so das sich die Dosierung des der Behandlungsflüssigkeit beigemischten Entkeimungsmittels überwachen und ggf. auch regeln läßt.

7. Einrichtung nach einem der Ansprüche 1 bis 6, **dadurch gekennzeichnet, dass** dem Vorratsbehälter eine Dosiervorrichtung für mindestens ein Entkeimungsmittel zugeordnet ist, so dass das Entkeimungsmittel der Behandlungsflüssigkeit im Vorratsbehälter beigemischt wird.

8. Einrichtung nach einem der Ansprüche 1 bis 7, **dadurch gekennzeichnet, dass** dem Vorratsbehälter (6) eine Entkeimungsanlage (5) vorgeschaltet ist.

9. Einrichtung nach einem der Ansprüche 1 bis 8, **dadurch gekennzeichnet, daß** ein Ablaßventil (15) vorgesehen ist, über das sich der Vorratsbehälter (6) direkt entleeren läßt.

10. Einrichtung nach einem der Ansprüche 1 bis 9, **dadurch gekennzeichnet, dass** mehrere Handstücke des medizinischen Gerätes parallel über zugeordnete Anschlußventile (4) an die Zuleitung (2) für die Behandlungsflüssigkeit angeschlossen sind und dass die von den Umschaltventilen (9) der einzelnen Handstücke ausgehenden Rückspülleitungsabschnitte (10a) in eine gemeinsame Rückspülleitung (10) münden.

11. Einrichtung nach Anspruch 10, **dadurch gekennzeichnet, daß** jedem Handstück ein eigenes Rückspülventil zugeordnet ist.

12. Einrichtung nach Anspruch 10, **dadurch gekennzeichnet, daß** in der gemeinsamen Rückspülleitung (10) ein gemeinsames Rückspülventil (11) für alle angeschlossenen Handstücke vorgesehen ist.

## Claims

1. A system for feeding treatment liquid into medical apparatus having a discharge aperture for the treatment liquid, particularly for feeding water into dental equipment, comprising
• a feed pipe (2) for the treatment liquid,
• means for introducing germicide into the treatment liquid, and
• a switch valve (9) disposed upstream of the discharge aperture, to which switch valve the feed pipe (2) is connected,
• a reservoir (6) being provided for the treatment liquid, from which reservoir (6) the treatment liquid is transported with the aid of a pump (7),
**characterized in that** a backwash pipe (10) containing a backwash valve (11) is connected to the switch valve (9) and that the backwash pipe (10) is capable of being alternately connected via a discharge valve (12) to an outflow drain (13) or to the reservoir (6) so that the treatment liquid can be alternately pumped into the outflow drain (13) or into the circuit comprising the reservoir (6) through the feed pipe (2) leading to the switch valve (9) and back into the reservoir (6) through the backwash pipe (10).

2. A system as defined in claim 1, **characterized in that** the switch valve (9) and the backwash valve (11) are constructed to form a three-way valve disposed upstream of the discharge aperture.

3. A system as defined in claim 1, **characterized in that** the backwash valve (11) is disposed in the backwash pipe (10) and that the switch valve (9) is pressure-controlled such that, when the backwash valve (11) is closed, the switch valve (9) opens by reason of the stagnation pressure forming in the backwash pipe (10) and closes when the backwash valve (11) is opened.

4. A system as defined in any one of claims 1 to 3, **characterized in that** the switch valve (9) is in the form of a check valve.

5. A system as defined in any one of claims 1 to 4, **characterized in that** means are provided for determining the type and/or amount of germs present so that the type and amount of germicide to be added and the backwash time can be individually specified.

6. A system as defined in any one of claims 1 to 5, **characterized in that** means are provided for determining the concentration and possibly the type of germicide present in the treatment liquid so that the rate at which the germicide is added to the treatment liquid can be monitored and regulated when necessary.

7. A system as defined in any one of claims 1 to 6, **characterized in that** a dosing mechanism for at least one germicide is associated with the reservoir to enable the germicide to be added to the treatment liquid in the reservoir.

8. A system as defined in any one of claims 1 to 7, **characterized in that** a sterilization device (5) is disposed upstream of the reservoir (6).

9. A system as defined in any one of claims 1 to 8, **characterized in that** a discharge valve (15) is provided, through which the reservoir (6) can be directly emptied.

10. A system as defined in any one of claims 1 to 9, **characterized in that** several hand instruments of the medical apparatus are connected in parallel through associated connecting valves (4) to the feed pipe (2) for the treatment liquid and that the sections (10a) of the backwash pipe leading from the switch valves (9) of the individual hand instruments are connected to a common backwash pipe (10).

11. A system as defined in claim 10, **characterized in that** each hand instrument is provided with its own backwash valve.

12. A system as defined in claim 10, **characterized in that** in the common backwash pipe (10) there is provided a common backwash valve (11) for all connected hand instruments.

## Revendications

1. Dispositif destiné à introduire du liquide de traitement dans des appareils médicaux, comprenant une ouverture de sortie pour le liquide de traitement, notamment pour l'introduction d'eau dans des dispositifs dentaires,
- avec une conduite d'amenée (2) pour le liquide de traitement,
- avec des moyens pour l'apport d'agents désinfectants dans le liquide de traitement et
- avec une soupape d'inversion (9) montée avant l'ouverture de sortie, à laquelle est raccordée la conduite d'amenée (2),
- un réservoir (6) pour le liquide de traitement étant prévu et le liquide de traitement étant refoulé à l'aide d'une pompe (7) hors du réservoir (6),
**caractérisé en ce qu'**une conduite de rinçage à contre-courant (10) avec une soupape de rinçage à contre-courant (11) est raccordée à la soupape d'inversion (9) et **en ce que** la conduite de rinçage à contre-courant (10) peut être raccordée par le biais d'une soupape d'évacuation (12) soit à une évacuation (13) soit au réservoir (6), de sorte que le liquide de traitement soit pompé dans l'évacuation (13) ou soit pompé dans le circuit du réservoir (6) par le biais de la conduite d'amenée (2) à la soupape d'inversion (9) et par le biais de la conduite de rinçage à contre-courant (10) à nouveau dans le réservoir (6).

2. Dispositif selon la revendication 1, **caractérisé en ce que** la soupape d'inversion (9) et la soupape de rinçage à contre-courant (11) sont réalisées dans une soupape à trois voies montée en amont de l'ouverture de sortie.

3. Dispositif selon la revendication 1, **caractérisé en ce que** la soupape de rinçage à contre-courant (11) est disposée dans la conduite de rinçage à contre-courant (10) et **en ce que** la soupape d'inversion (9) est commandée en pression, de sorte qu'elle s'ouvre sous l'effet de la pression dynamique augmentant dans la conduite de rinçage à contre-courant (10) lorsque la soupape de rinçage à contre-courant (11) est fermée, et qu'elle se ferme lorsque la soupape de rinçage à contre-courant (11) est ouverte.

4. Dispositif selon l'une quelconque des revendications 1 à 3, **caractérisé en ce que** la soupape d'inversion (9) est réalisée en tant que clapet anti-retour.

5. Dispositif selon l'une quelconque des revendications 1 à 4, **caractérisé en ce que** l'on prévoit des moyens pour déterminer le type et/ou la quantité des germes présents de sorte que le type et le dosage de l'agent désinfectant à mélanger ainsi que la durée du rinçage à contre-courant puissent être déterminés individuellement.

6. Dispositif selon l'une quelconque des revendications 1 à 5, **caractérisé en ce que** l'on prévoit des moyens pour déterminer la concentration et éventuellement le type d'agent désinfectant dans le liquide de traitement, de sorte que le dosage de l'agent désinfectant mélangé au liquide de traitement puisse être surveillé et éventuellement aussi régulé.

7. Dispositif selon l'une quelconque des revendications 1 à 6, **caractérisé en ce que** l'on associe au réservoir un dispositif de dosage pour au moins un agent désinfectant, de sorte que l'agent désinfectant soit mélangé au liquide de traitement dans le réservoir.

8. Dispositif selon l'une quelconque des revendications 1 à 7, **caractérisé en ce qu'**une installation de désinfection (5) est montée avant le réservoir (6).

9. Dispositif selon l'une quelconque des revendications 1 à 8, **caractérisé en ce que** l'on prévoit une soupape d'évacuation (15) par le biais de laquelle on peut vider directement le réservoir (6).

10. Dispositif selon l'une quelconque des revendications 1 à 9, **caractérisé en ce que** plusieurs pièces manuelles de l'appareil médical sont raccordées parallèlement, par le biais de soupapes de raccord associées (4), à la conduite d'amenée (2) pour le liquide de traitement et **en ce que** les portions de la conduite de rinçage à contre-courant (10a) partant depuis les soupapes d'inversion (9) des pièces manuelles individuelles débouchent dans une conduite de rinçage à contre-courant (10) commune.

11. Dispositif selon la revendication 10, **caractérisé en ce que** l'on associe à chaque pièce manuelle sa propre soupape de rinçage à contre-courant.

12. Dispositif selon la revendication 10, **caractérisé en ce que** l'on prévoit dans la conduite de rinçage à contre-courant (10) commune une soupape de rinçage à contre-courant (11) commune pour toutes les pièces manuelles raccordées.
